(19) Europäisches Patentamt
European Patent Office
Office européen des brevets

(11) EP 2 111 879 A1

(12) # EUROPÄISCHE PATENTANMELDUNG

(43) Veröffentlichungstag:
**28.10.2009 Patentblatt 2009/44**

(51) Int Cl.:
***A61L 27/60*** (2006.01) ***C12N 5/00*** (2006.01)

(21) Anmeldenummer: **08169840.9**

(22) Anmeldetag: **25.11.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **25.01.2008 DE 102008006086**

(71) Anmelder: **Evonik Goldschmidt GmbH**
**45127 Essen (DE)**

(72) Erfinder:
- **Scheuermann, Ralph, Dr.**
  **45359 Essen (DE)**
- **Frech, Christian, Prof. Dr.**
  **68219 Mannheim (DE)**
- **Meyer, Jürgen, Dr.**
  **45134 Essen (DE)**

(54) **Biomimetische Modellsysteme zur Nachstellung von Spreitungsphänomenen kosmetischer und pharmazeutischer Formulierungen auf menschlicher Haut**

(57) Die Erfindung betrifft einen Hautersatzstoff zur Untersuchung des Verhaltens von topisch anzuwendenden, kosmetischen und pharmazeutischen Kompositionen, wobei der Hautersatzstoff eine dreidimensional vernetzte Polyacrylamid-Matrix ist und in besagter Matrix Wasser und mindestens ein Polypeptid eingebunden ist.



Printed by Jouve, 75001 PARIS (FR)

## Beschreibung

Gebiet der Erfindung

Die Erfindung betrifft eine Vorrichtung zur Simulation der menschlichen Hautoberfläche, um an dieser Vorrichtung physikalische Phänomene wie das Spreitungsverhalten von Ölen nachstellen und untersuchen zu können

Für topisch zu applizierende kosmetische und pharmazeutische Formulierung ist das Verhalten beim Auftragen auf die Haut von entscheidender Wichtigkeit. Eine oft gemessene Größe ist hier die Spreitungsgeschwindigkeit der zu testenden Substanz.

Um repräsentative Messdaten zu erhalten, werden in der Praxis nach wie vor sogenannte Paneltests an humanen Probanden durchgeführt. Diese Tests sind teuer, arbeitsam und außerdem bedarf es aufgrund der hohen Varianz an Individualparametern wie Hautfeuchtigkeit, Oberflächenbeschaffenheit etc. einer sehr großen Anzahl an Probanden, um reproduzierbares Datenmaterial zu erhalten. Der Bedarf nach einem neuen Modellsystem, mit dem sich preiswert und reproduzierbar die Ergebnisse der Paneltests im Labor abbilden lassen, also einem Mimetikum für menschliche Haut, ist daher groß.

Haut ist aufgrund der unzähligen hohen an sie gestellten Anforderungen ein komplex aufgebautes Gewebe bestehend aus mehreren Schichten verschiedener Zelltypen. Die Zellwände wiederum sind durchsetzt mit komplexen Mischungen verschiedenster chemischer Substanzen wie z. B. Ceramiden, Phospolipiden und Fettsäuren. Diese Tatsache erschwert die Auswahl geeigneter Stoffe zur Nachbildung der menschlichen Hautoberfläche.

Stand der Technik

Im Stand der Technik sind Modelle aus acrylatischen Polymeren beschrieben, die zum Testen zahlreicher Eigenschaften kosmetischer Produkte geeignet sein sollen, so z.B. Bestimmung der Diffusion toxischer Substanzen in die Matrix hinein, Ermittelung von Sonnenschutzfaktoren, Untersuchung der Verteilungseigenschaften und Verhalten fester Make-up-Partikel (WO9721097).

US-Patentschrift 4877454 beschreibt ein Hautmodell zum Testen des Klebeverhaltens von Pflastern, welches auf der Benutzung von Gelatine-Polymer-Matrizes basiert. Gelatine eignet sich nicht für die Bestimmung von Spreitgeschwindigkeiten, da die Reproduzierbarkeit der Ergebnisse aufgrund der Inhomogenität der Gelatine-Polymerisation nicht gegeben ist. Der Polymerisationsprozess führt zu uneinheitlichen Porengrößen, sowohl innerhalb einer Matrix als auch verglichen zwischen zwei unabhängigen Polymerisationsvorgängen. Hinzu kommt die Chargen-Varianz, die wie bei allen biologischen Materialien wie Gelatine die Reproduzierbarkeit weiter beeinträchtigt. Außerdem spreiten einige Substanzen auf Gelatineplatten zudem sehr unregelmäßig (Ausbildung von Spreitfingern).

Dieselben Nachteile ergeben sich bei dem in US Patent 5015431 beschriebenen Modell, welches ebenfalls für die Untersuchung von Klebstoffen entwickelt wurde und auf polymerisierbaren, löslichen Proteinen basiert.

Im Stand der Technik finden sich keine Informationen darüber, wie man reproduzierbar die in einem Paneltest gefundene Geschwindigkeitsreihe der durchschnittlichen Spreitgeschwindigkeiten verschiedener Substanzen in einem in-vitro-Modell nachbilden kann.

Aufgabe der vorliegenden Erfindung war es daher, ein alternatives Material bereitzustellen, mit dem das Spreitverhalten von Substanzen auf der Haut reproduzierbar simuliert bzw. vorhergesagt werden kann und auf dem die Ausbildung von Spreitfingern nicht zu beobachten ist. Des Weiteren sollte ein Material entwickelt werden, dessen Oberflächenbeschaffenheit in der Form variabel gestalten werden kann, so dass reproduzierbar die in einem Paneltest gefundene Geschwindigkeitsreihe der durchschnittlichen Spreitgeschwindigkeiten verschiedener Substanzen in einem in-vitro-Modell nachgebildet werden können.

Beschreibung der Erfindung

Überraschenderweise wurde gefunden, dass eine Vorrichtung, die einen Hautersatzstoff zur Nachahmung der physikalischen Oberflächeneigenschaften der menschlichen Haut zur Untersuchung des Verhaltens von topisch anzuwendenden, kosmetischen und/oder pharmazeutischen Produkte, wobei der Hautersatzstoff eine dreidimensional vernetzte Polyacrylamid-Matrix ist, die Oberflächenbeschaffenheit bezüglich des Spreitverhaltens der menschlichen Haut vorzüglich nachbildet, wenn in der Matrix Wasser und mindestens ein Polypeptid eingebunden ist.

Gegenstand der vorliegenden Erfindung sind deshalb Vorrichtungen wie in Anspruch 1 beschrieben, ein Verfahren wie in Anspruch 10 beschrieben sowie die Verwendung der Vorrichtung wie in Anspruch 11 beschrieben.

Durch die Erfindung kann nun schnell und kostensparend die optimale Zusammensetzung einer topischen Formulierung in mehreren z. B. Wirkstoff-Verdünnungs- oder Emollient-Konzentrationsreihen ermittelt werden. Eine solch qualitativ vergleichende in-vitro-Simulation von Paneltestergebnissen ist mit bisherigen Vorrichtungen nach dem Stand der Technik wegen oben aufgeführter Nachteile nur schlecht oder gar nicht möglich.

Das erfindungsgemäße System hat außerdem den Vorteil, dass es die Möglichkeit bietet, die zu erhaltenden in-vitro-Ergebnisse in der Art zu beeinflussen, dass diese den aus einem vorangegangenen Paneltest hervorgegangenen Referenzwerten unterschiedlicher Testsubstanzen möglichst nahekommen.

Die erfindungsgemäßen Hautmimetika und das Verfahren zur Angleichung dieser für unterschiedliche Testemulsionen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören.

Die erfindungsgemäße Vorrichtung umfassend einen Hautersatzstoff (zur Nachahmung der physikalischen Oberflächeneigenschaften der menschlichen Haut) zur Untersuchung des Verhaltens von topisch anzuwendenden, kosmetischen und/oder pharmazeutischen Produkte, wobei der Hautersatzstoff eine dreidimensional vernetzte Polyacrylamid-Matrix ist, zeichnet sich dadurch aus, dass in besagter Matrix Wasser und mindestens ein Polypeptid eingebunden ist.

*Topisch anzuwendende, kosmetische oder pharmazeutische Kompositionen*

Unter topisch anzuwendenden, kosmetischen oder pharmazeutischen Kompositionen sind im Rahmen der Erfindung kosmetische oder pharmazeutische Formulierungen so wie einzelne oder mehrere Bestandteile von solchen Formulierungen zu verstehen. Einzelne Bestandteile sind beispielsweise Emollients, Emulgatoren, Tenside, Verdicker, Viskositätsregler, Stabilisatoren, UV-Lichtschutzfilter, Antioxidantien, Hydrotrope und Polyole, Fest- und Füllstoffe, Filmbildner, Perlglanzadditive, Deodorant- und Antitranspirantwirkstoffe, Insektrepellentien, Selbstbräuner, Konservierungsstoffe, Konditioniermittel, Parfüme, Farbstoffe, biogene Wirkstoffe, Pflegeadditive, Überfettungsmittel oder Lösungsmittel.

*Polyacrylamid*

Als Polyacrylamid-Matrix kann prinzipiell jede bekannte dreidimensional vernetzte Polyacrylamid-Matrix verwendet werden. Dreidimensional vernetzte Polyacrylamid-Matrizen sind dem biochemischen Fachmann hinlänglich bekannt, da sie z. B. während der Laborroutine in Form von Gelen zur Trennung von Protein-und DNA-Mischungen verwendet werden.

Geeignete Polyacrylamid-Matrizen können z. B. durch Polymerisation von Acrylamid in wässriger Lösung in der Anwesenheit von kleinen Mengen eines mindestens bifunktionalen Quervernetzers (Crosslinker) gebildet werden. Als Crosslinker können eine oder mehrere Verbindungen ausgewählt aus z.B. N,N'-Methylenbisacryl-amid (MBA), Piperazin-Diacrylat, N,N'-Bisacrylylcystamin und N,N'-Diallyltartatdiamid eingesetzt werden. Vorzugsweise wird als Crosslinker MBA eingesetzt. Durch die Copolymerisation von Acrylamid und MBA kann ein gitterartiges Netzwerk in drei Dimensionen, das aus Acrylamidketten mit Zwischenverbindungen, die durch das MBA entstanden sind, geformt werden.

Die Herstellung von geeigneten Polyacrylamid-Matrizes wird z.B. in Sambrook et al. Molecular Cloning: A Laboratory Manual. 2nd ed., Cold Spring Harbor Laboratory, 1989 beschrieben.

Die Zusammensetzung der Polyacrylamid-Matrizes wird üblicherweise durch eine Nomenklatur beschrieben, die das Verhältnis von Acrylamid zu Quervernetzer, wie z.B. Bisacylamid, zu Wasser beschreibt. In dieser Nomenklatur steht T für den prozentualen Anteil (weight per volume; w/v) der Summe von Acrylamid und Quervernetzer zusammen in wässriger Lösung und C für den prozentualen Massenanteil (weight per weight; w/w) von Crosslinker-Monomeren an der Summe aus Crosslinker-Monomeren und Acrylamid-Monomeren. Eine fünf (w/v) prozentige wässrige Polyacrylamid-Matrix, in welcher das Massenverhältnis MBA-Monomeren zu Acrylamid-Monomeren 1:33 entspricht, hätte dementsprechend Werte von T = 5% und C = 3%.

Durch Variation dieser Werte können je nach Bedarf Polyacrylamid-Matrizes mit unterschiedlichen Porengrößen erhalten werden. Die Porengröße der Polymermatrix kann verringert werden, indem der Wert T vergrößert wird. Die Porengröße kann auch durch die Variation von C eingestellt werden.

Erfindungsgemäße Vorrichtungen weisen vorzugsweise Polyacrylamid-Matrizes mit einem T-Wert von 3 bis 15 %, bevorzugt von 4 bis 12 %, besonders bevorzugt von 4 bis 8 % und besonders bevorzugt von 5 % auf.

Der C-Wert von erfindungsgemäß bevorzugten Polyacrylamid-Matrizes beträgt vorzugsweise von 2 bis 6 %, bevorzugt von 2 bis 4 % und besonders bevorzugt 3 %.

Besonders bevorzugte Vorrichtungen weisen Polyacrylamid-Matrizes mit T und C Werten von T = 3 bis 15 % und C = 2 bis 6 %, bevorzugt von T = 4 bis 12 % und C = 2 bis 4 %, und besonders bevorzugte von T = 5 % und C = 3 % auf. Ideal sind rehydratisierte Matrizes der Firma GE Healthcare "CleanGel™ for IEF".

*Polypeptide*

Als Polypeptide können in der erfindungsgemäßen Vorrichtung prinzipiell alle Polypeptide vorhanden sein. Die Familie der organisch chemischen Verbindungen "Polypeptide" ist dem biochemischen Fachmann als Substanzklasse hinlänglich bekannt. Die Polypeptide zeichnen sich dadurch aus, dass einzelne Aminosäuren über eine Amidbindung in einer definierten Reihenfolge zu einer verzweigten oder unverzweigten Kette verbunden sind. Beispiele für synthetische Polypeptide sind Polyornithin und Polylysin, ein Beispiel für ein verzweigtes, natürlich vorkommendes Polypeptid ist Cyanophycin.

Im Rahmen der vorliegenden Erfindung sollen unter dem Begriff "Polypeptid" auch längere Aminosäureketten (mehr als 100 Aminosäuren), die man allgemein als "Proteine" oder auch "Eiweiße" bezeichnet, verstanden werden. Eiweiße werden in unterschiedliche Gruppen aufgeteilt; die Gruppen der Albumine und Globuline zeichnen sich durch ihr abundantes Vorkommen und ihre einfache Gewinnung aus. Hierdurch sind Mitglieder dieser Gruppen wie z.B. Bovines Serum Albumin, Ovalbumin, alpha- und beta-LactoGlobulin meist kommerziell in großen Mengen preiswert erhältlich.

Vorzugsweise weisen erfindungsgemäße Vorrichtungen als Polypeptide Proteine ausgewählt aus der Familie der Globuline oder Albumine auf. Bevorzugt weisen erfindungsgemäße Vorrichtungen als Polypeptide Bovines Serum Albumin oder beta-Lactoglobulin, besonders bevorzugt beta-Lactoglobulin auf.

Bevorzugte Ausführungsformen der erfindungsgemäßen Vorrichtung weisen Polyacrylamid-Matrizes mit einem Peptidgehalt von 0,5 bis 5, vorzugsweise 1 bis 4 und besonders bevorzugt 2 bis 3 g Polypeptid pro Liter Matrixvolumen auf.

*pH-Wert*

Es kann vorteilhaft sein, wenn die Polyacrylamid-Matrizes einen pH-Wert von 4 bis 10, vorzugsweise 5 bis 8 und besonders bevorzugt einen pH-Wert von 5,5 aufweisen. Der pH-Wert kann durch Inkubation der Matrizes in Puffern beeinflusst werden. Zur Einstellung des pH-Werts weisen die Polyacrylamid-Matrizes vorzugsweise ein geeignetes Puffer-System, z. B. Tris, PBS oder Carbonat-Puffer, besonders bevorzugt Acetat-Puffer auf.

Den Polyacrylamid-Matrizes können weitere organische Substanzen wie z.B. Lipide oder Ceramide beigemengt werden.

Die Vorrichtung kann eine Kamera zur Dokumentation und/oder Messung der Spreitung anhand von durch die Kamera erhaltener Abbildungen aufweisen.

Bevorzugt besitzt die Vorrichtung eine CCD-Kamera, vorzugsweise mit einer CCD-Größe von 0,5'', 1392 x 1040 effektiven Pixeln und einer Auflösung von 12 bit, die an einen Rechner mit einer Auswertesoftware angeschlossen ist. Bevorzugte Auswertesoftware ist "Image J".

Zur exakten Auftragung der zu untersuchenden Substanz kann die Vorrichtung eine Spritze aufweisen. Bevorzugt weist die Vorrichtung eine Hamilton-Spritze auf. Besonders bevorzugt ist die Hamilton-Spritze in einer höhenverstellbaren Halterung angebracht.

Eine bevorzugte Vorrichtung ist **dadurch gekennzeichnet, dass** die für die automatische Auswertung notwendige Kontrastbildung durch Lichtbrechung innerhalb des Tropfens durch Anstrahlen eines weißen Ausschnittes in der Größe des Substrates innerhalb einer schwarzen Fläche unterhalb des Substrates mittels einer Hauptlichtquelle erzielt wird.

Die erfindungsgemäße Vorrichtung bzw. die Polyacrylamid-Matrix kann dadurch erhalten werden, dass eine geeignete Menge mindestens eines geeigneten Polypeptids in eine Polyacrylamid-Matrix eingebettet wird. Das Polypetid kann vor dem Polymerisationsprozess des Acrylamides homogen beigemischt werden und somit die Matrix gleichförmig durchziehen. Es kann kovalent an die Matrix gebunden sein. Das Polypeptid kann in die Polyacrylamid-Matrix eingebracht werden, indem die Matrix in das Polypeptid enthaltender wässriger Lösung inkubiert wird oder aber zuvor getrocknete Polyacrylamid-Matrizes in das Polypeptid enthaltender wässriger Lösung rehydriert werden.

Vorzugsweise erfolgt die Herstellung einer geeigneten Polyacrylamid-Matrix durch das erfindungsgemäße Verfahren zur Anpassung der erfindungsgemäßen Vorrichtung an eine Reihe zu untersuchender Substanzen, welches sich durch die Verfahrensschritte A. Bestimmung der Parameter $C_1$ und n des Spreitverhaltens der zu untersuchenden Kompositionen im Rahmen eines Paneltests, B. variieren der C- und T-Werte der Polyacrylamid-Matrix, des Polypeptides und/oder seiner Konzentration und/oder des pH-Wertes und/oder der Art des Einbringens des Polypeptides in die Matrix unter Erhalt verschiedener Ausführungsformen und Bestimmung der Parameter $C_1$ und n des Spreitverhaltens der zu untersuchenden Kompositionen für jede Ausführungsform, und C. vergleichen der Parameter aus A. und B. und Auswahl der Ausführungsform der Vorrichtung mit der Polyacrylamid-Matrix, die die größte Korrelation der Messdaten zeigt, auszeichnet.

*Mathematisches Kinetikmodell für Spreitungsverhalten*

Zur Auswertung der experimentellen Daten wurde ein mathematisches Modell der Spreitkinetik eines Tropfens auf einer Oberfläche entwickelt. Die Formel:

$$A(t) = C_1 * t^n$$

gibt die Oberfläche (A) eines spreitenden Tropfens moduliert durch die Parameter $C_1$ und n in Abhängigkeit der Zeit (t) wieder.
Logarithmieren ergibt

$$\log A = n \log t + \log C_1$$

Bei experimentell ermitteltem A und t konnte durch doppelt logarithmische Darstellung der Messdaten in Excel (Microsoft Corp.) nach der allgemeinen Geradengleichung

$$y = mx + b$$

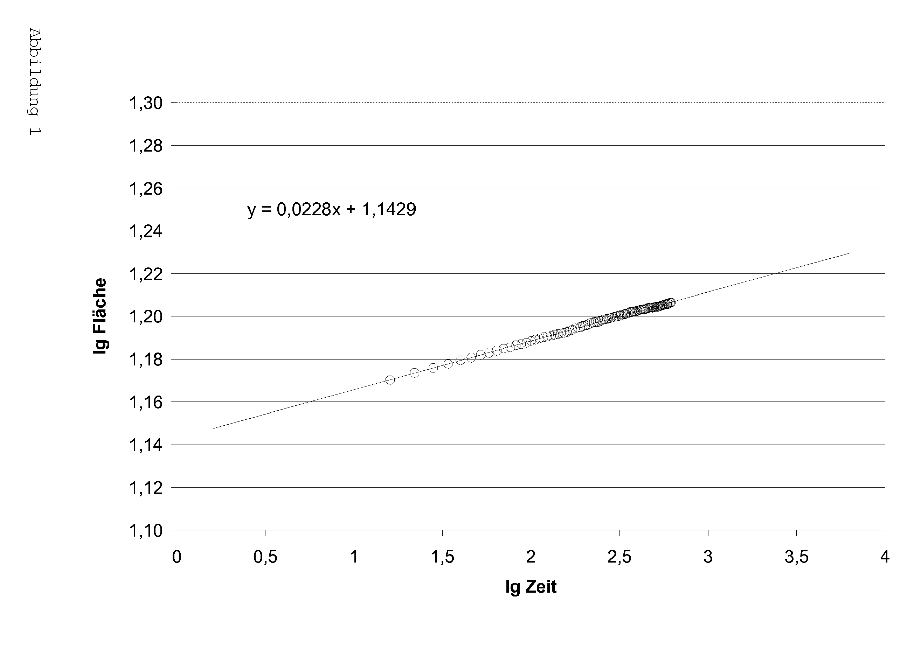

über den Achsenabschnitt und die Steigung $C_1$ und n berechnet werden.
Das mathematische Modell gab die gemessen Werte gut wieder.
Vergleiche exemplarisch Abbildung 1.
Ein erfindungsgemäßes Verfahren, um die Vorrichtung an unterschiedliche, zu vermessende Substanzen $X_1$ bis $X_y$ optimal anzupassen beinhaltet vorzugsweise: Bestimmung der Parameter $C_1$ und n gemäß "Mathematische Kinetikmodell" des Spreitverhaltens der Substanzen $X_1$ bis $X_y$ im Rahmen eines Paneltests; Variieren der C- und T-Werte der Polyacrylamid-Matrix, des Polypeptides und seiner Konzentration und des pH-Wertes (Ausführungsform a1 bis an) und Bestimmung der Parameter $C_1$ und n für jede Ausführungsform $a_1$ bis an für die Substanzen $X_1$ bis $X_y$ und ein Vergleichen der Parameter $C_1$ und n für ($X_{1PanelTest}$ bis $X_{yPaneltest}$) mit den jeweiligen für ($X_{1a1}$ bis $X_{ya1}$) bis ($X_{1an}$ bis $X_{yan}$) ermittelten und anschließender Wahl des best geeigneten Zusammensetzung der Vorrichtung (Ausführungsform $a_{opt}$) durch Korrelation der Messdaten.

*Paneltest*

In dem Paneletest wird vorzugsweise eine möglichst große Anzahl an Probanden eingesetzt. Bevorzugt werden mehr als 10, besonders bevorzugt mehr als 15 Personen eingesetzt. Die Probanden können jeglichen Geschlechts und aus jeglicher Altersklasse sein. Bevorzugt repräsentiert die Gruppe an Probanden statistisch bezüglich Geschlecht und Alter die Durchschnittsbevölkerung Europas.
Bei den Probanden können jegliche Hautstellen zur Untersuchung des Spreitverhaltens eingesetzt werden. Bevorzugt wird die Innenseite des Unterarmes eingesetzt. Die Hautpartie kann vor Applikation des Testsubstanz gereinigt werden. Bevorzugt wird diese Partie mit einer 1%igen Betainlösung und anschließend mit klarem Wasser gewaschen. Es kann vorteilhaft sein, eine Grössenmarkierung an der eingesetzten Hautpartie anzubringen. Bevorzugt wird mit Stempelfarbe eine kreisförmige Markierung von 5 cm Durchmesser aufgetragen. Eine kleine Menge, bevorzugt 10 µL der zu untersuchenden Substanz werden in der Mitte der Markierungen aufgetragen. Das Auftragen erfolgt vorzugsweise mit einer Mikropipette. Die Längsachsen der resultierenden Ellipsen werden zu unterschiedlichen Zeitpunkten bestimmt. Bevorzugt wird für diese Grössenbestimmung eine Schieblehre eingesetzt. Die Bestimmung erfolgt vorzugsweise nach 3 und 5 Minuten oder nach 5 und 15 Minuten.

*Simulation*

Zur Simulation von Spreitungsphänomenen kosmetischer und pharmazeutischer Formulierungen mit einem biomimetischen Modellsystem für menschliche Haut wir die zu untersuchende Substanz möglichst punktförmig auf die Polyacrylamid-Matrix aufgetragen. Hierzu wird bevorzugt eine Hamiltonspritze, gefüllt mit der Testsubstanz, eingesetzt. Diese wird möglichst nah, vorzugsweise mit Hilfe einer höhenverstellbaren Halterung und bevorzugt auf 0,6 mm Abstand, der Polyacrylamid-Matrix angenähert und eine kleine Menge, bevorzugt 10 μL, werden auf die Matrix appliziert.

Nach Aufgabe des Tropfens und gegebenenfalls Entfernen der Spritze kann das Spreitverhalten protokolliert werden. Bevorzugt wird mittels einer Kamera die Änderungen am System verfolgt, bevorzugt mit einem digitalen Kamerasystem, besonders bevorzugt wird eine NET Foculus 432Sc/B eingesetzt.

Die für die automatische Auswertung notwendige Kontrastbildung kann durch Lichtbrechung innerhalb des Tropfens durch Anstrahlen eines weißen Ausschnittes in der Größe des Substrates innerhalb einer schwarzen Fläche unterhalb des Substrates mittels einer Hauptlichtquelle erzielt werden.

Die Fläche des Tropfens auf dem Substrat kann anhand der Bilder, die zu unterschiedlichen Zeitpunkten aufgenommen wurden, bestimmt werden. Die Auswertung der Bilder kann mithilfe einer Software, bevorzugt das Programms "Image J" erfolgen.

Das erfindungemäße Verfahren kann bei beliebiger Temperatur und Luftfeuchtigkeit durchgeführt werden; bevorzugt werden alle Messungen in einem Klimaraum, besonders bevorzugt bei einer Temperatur von 24,5 ± 0,5 °C und einer Luftfeuchtigkeit von 55 ± 5% durchgeführt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele

*Mikroskop Aufbau/ Messverfahren*

Nach Aufgabe von 10 μl des Emollients mittels einer Hamilton-spritze, welche in eine justierbare Halterung eingespannt war, wurde mittels einer Kamera (NET Foculus 432Sc/B) alle 6 Sekunden ein Bild vom Tropfen auf dem Substrat aufgenommen. Die Fläche des Tropfens auf dem Substrat wurde bestimmt. Dazu wurden die Bilder mithilfe des Programms "Image J" ausgewertet, indem das Programm die Fläche innerhalb des in der Regel kreisförmigen Randes des Tropfens als Pixelzahl berechnete. Die Fläche wurde durch Vergleich mit einem Objekt bekannter Größe in $mm^2$ umgerechtet. Für die automatische Auswertung war es notwendig, daß die Konturen des Tropfens sich kontrastreich vom transparenten Substrat abhoben. Für die erforderliche Lichtbrechung innerhalb des Tropfens wurde mit einer Hauptlichtquelle (hierfür diente eine einfache Energiesparlampe in ca. 30 cm Entfernung neben dem Substrat) eine schwarze Fläche (ca. 20 cm unterhalb des Substrates) mit einem weißen Ausschnitt in der Größe des Substrates angestrahlt. Die Reflexion des Lichtes erfolgte somit ausschließlich senkrecht zum Substrat, so daß es innerhalb des Tropfens durch die plankonvexe Form nicht zu einer Parallelverschiebung der Lichtstrahlen wie bei Durchdringung des planen Substrates, sondern zu einer veränderten Ausbreitungsrichtung des Lichtes und damit zur Lichtbrechung kam. Der Tropfen erschien somit für die Kamera schwarz. Die technischen Daten der Kamera sind wie folgt:

| CCD-Größe | 1/2" |
|---|---|
| effektive Pixel | 1392 x 1040 |
| Auflösung | 12 bit / 8 bit (= 4096 bzw. 256 Graustufen) |
| optischer Anschluß | C-Mount |
| Modi | Mono8, Mono16 |
| Digitales Interface | Firewire (IIDC 1.30) |
| Transfer Raten | 100 Mb/s |
| Vollbilder/sec | 20 (8 Bit), 10 (12 Bit) |
| Gain | manuell, 0 ... 25 db, Auto Gain |
| Scanning Modus | progressive scan |
| Verschluß | 1 μs ... 65 s, manuell/automatisch |
| Trigger | Software oder externer Trigger, Modi 0~5, 14, Einzelbilder o. Bildserien |
| Features | 1x2, 2x2 Binning, Blitzausgang |
| Gamma | 1.0 |
| Spannung | DC 8V ... 30 V über Firewire-Kabel |

(fortgesetzt)

| Gewicht | 110g |
|---|---|
| Abmessungen | 44mm x 29mm x 63mm |

*Herstellung der Gele*

Die bei -20 °C gelagerten Gele (CleanGel IEF) wurden vor Verwendung eine Stunde lang in einer Puffer-Lösung rehydratisiert. Die Pufferlösung enthielt 0,1% 100%ige Essigsäure und 0,8% Kaliumacetat (99%). Der Puffer wurde durch Zugabe von 5 molarer KOH-Lösung (85%) auf einen pH-Wert von 5,5 eingestellt.

Gegebenfalls wurde Protein wie z.B. BSA in den angegebenen Konzentrationen im Puffer gelöst. Die Puffer-Lösung wurde in einen GelPool gefüllt und ein Gelstück von etwa 1,5 x 1,5 cm Größe für eine Stunde eingelegt. Nach Entnahme des Gels wurde mit Hilfe eines Filterpapiers das Wasser von der Polyester-Seite entfernt und durch Aufstellen des Gelstückes auf eine Kante auf Filterpapier weiter getrocknet. Nach Ablauf einer 20-minütigen Trocknungszeit wurde die Messung gestartet.

*Reihe 0*

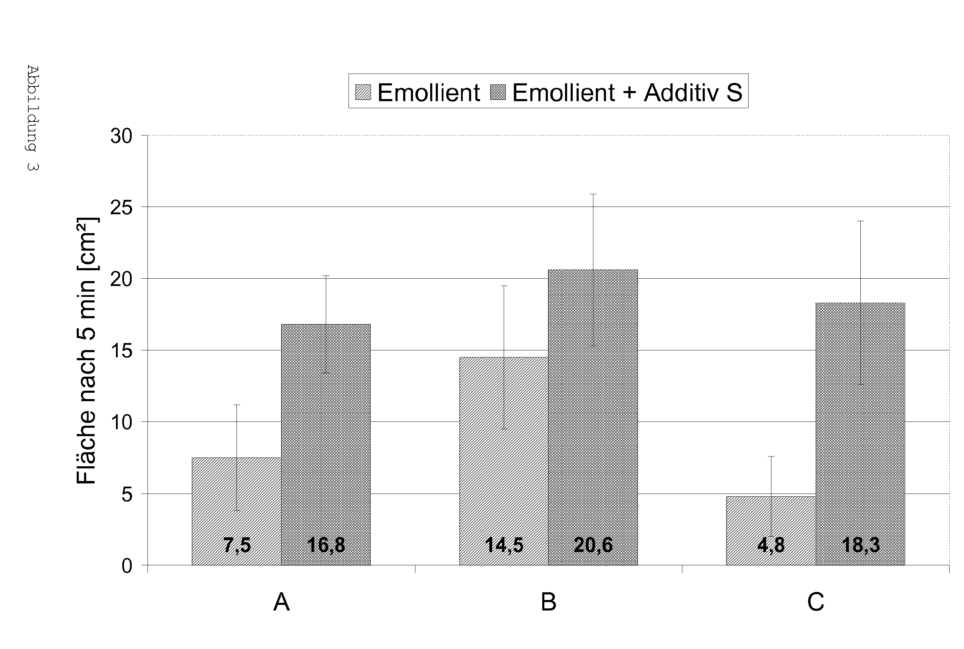

Der Paneltest wurde wie beschrieben mit drei Emollients - Decylcocoat (TEGOSOFT DC®), Bis-2-ethylhexyl-carbonat (TEGOSOFT DEC®), $C_{12}$-$C_{15}$-Alkylbenzoat (TEGOSOFT TN®), im weiteren mit A, B und C bezeichnet - sowie mit 0,1%igen Mischungen von Cetyldimethicone (ABIL WAX 9840®) in den genannten Emollients - im weiteren mit A + S, B + S und C + S bezeichnet, durchgeführt.
Der Vergleich der Spreitung erfolgte durch Mittelwertbildung der 5-Minutenwerte.
Als Ergebnis wurden für die Spreitung wie in Abbildung 3 dargestellt folgende Resultate gefunden:

- Für die Emollients gilt für die Spreitung folgende Reihenfolge: C < A < B.
- Für die Emollient-Cetyldimethiconemischungen gilt für die Spreitung: A < A + S, B < B + S und C < C + S. Weiterhin gilt folgende Reihenfolge: A + S ≤ C + S < B + S.
- Weiterhin gilt, dass die Spreitung von Emollient B als einziges in der Größenordnung der übrigen Emollients liegt.

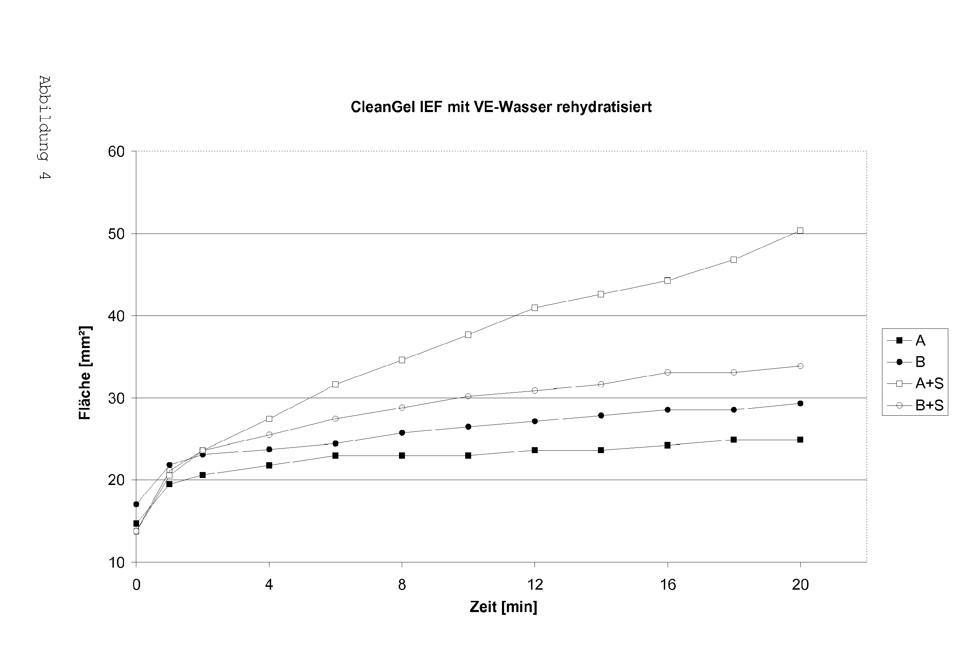

Die Spreitung der Emollients sowie der Emollient-Cetyldimethiconemischungen wurde wie beschrieben auf CleanGel IEF, welches mit VE-Wasser rehydratisiert worden war, durchgeführt. Für die Spreitung der Substanzen wurden wie in Abbildung 4 dargestellt folgende Resultate gefunden:

- Für die Emollients gilt für die Spreitung folgende Reihenfolge: A < B.
- Für die Emollient-Cetyldimethiconemischungen gilt für die Spreitung: A < A + S und B < B + S. Weiterhin gilt folgende Reihenfolge: B + S < A + S.

Mit CleanGel IEF rehydratisiert in VE-Wasser konnten die Ergebnisse des Paneltests somit nicht nachgestellt werden.

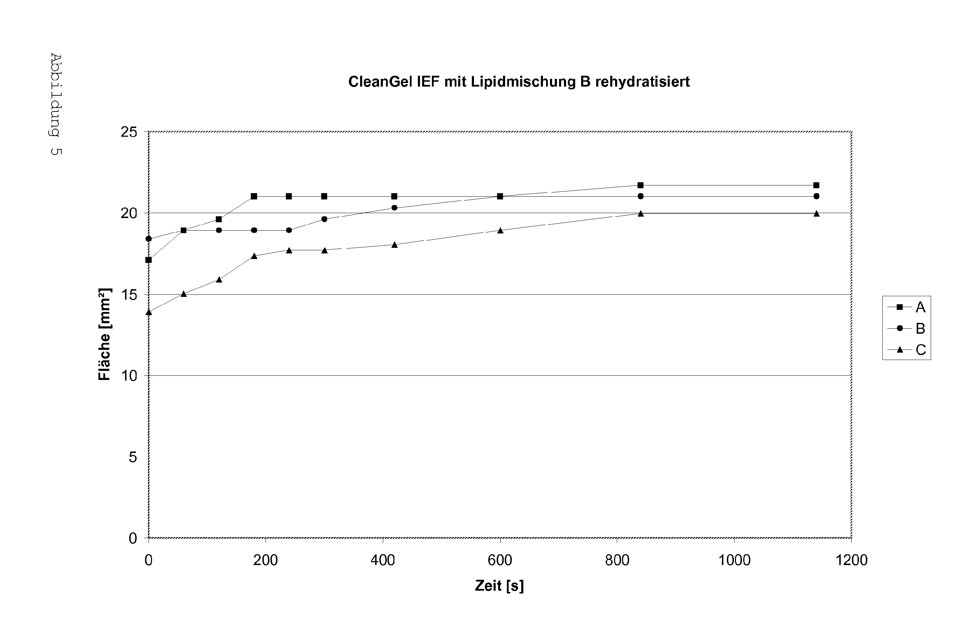

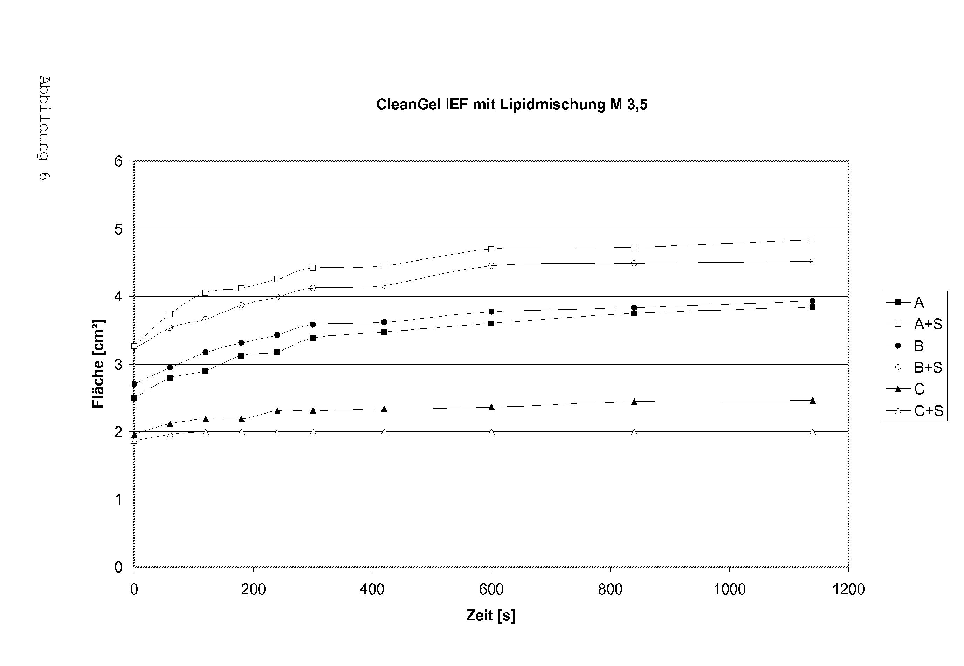

Die Spreitung der Emollients sowie der Emollient-Cetyldimethiconemischungen wurde wie beschrieben auf CleanGel IEF, welches a) mit einer Pflanzenöl/Wasseremulsion (1.5% (v/v) Sonnenblumenöl, welches durch zweimaliges Durchlaufen eines "French press cell disruption system" der Thermo Electron Corporation mit 1000 psi eingearbeitet wurde)und b) mit einer Protein/Lipidemulsion (H-Milch mit einem Fettgehalt von 3,5% (v/v)) rehydratisiert worden war, durchgeführt. Für die Spreitung der Substanzen wurden wie in Abbildung 5 und Abbildung 6 dargestellt folgende Resultate gefunden:

a)

- Für die Emollients gilt für die Spreitung folgende Reihenfolge: C < B < A

b)

- Für die Emollients gilt für die Spreitung folgende Reihenfolge: C < A < B
- Für die Emollient-Cetyldimethiconemischungen gilt für die Spreitung: A < A + S und B < B + S und C > C + S. Weiterhin gilt folgende Reihenfolge: C + S < B + S < A + S

Mit CleanGel IEF rehydratisiert in VE-Wasser, in Pflanzenöl-Emulsion und Milch konnten die Ergebnisse des Paneltests somit nicht nachgestellt werden.

*Reihe 1*

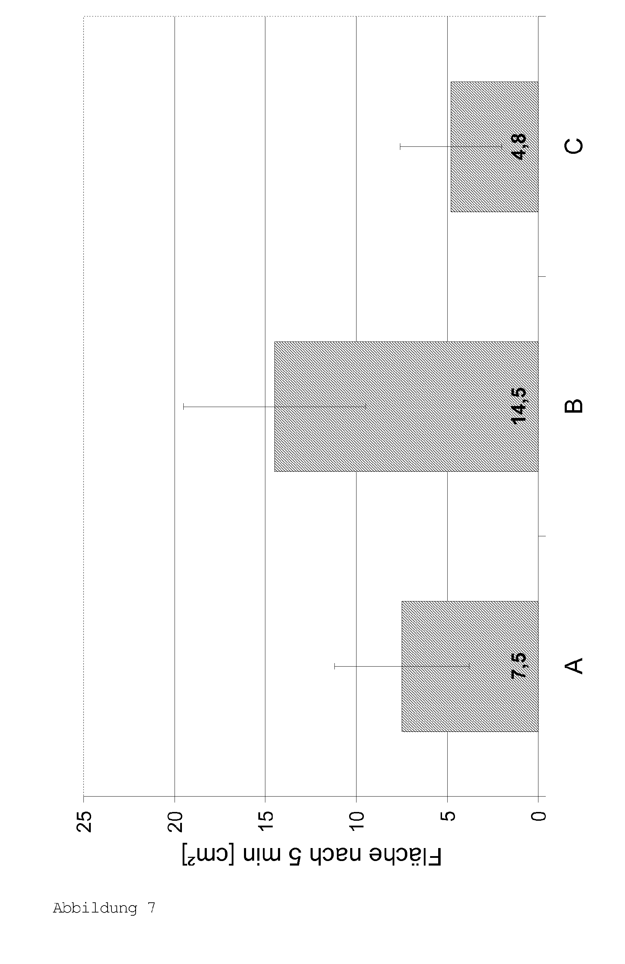

Der Paneltest wurde wie beschrieben mit drei Emollients - Decylcocoat (TEGOSOFT DC®), Bis-2-ethylhexylcarbonat (TEGOSOFT DEC®), $C_{12}$-$C_{15}$-Alkylbenzoat (TEGOSOFT TN®), im weiteren mit A, B und C bezeichnet - durchgeführt. Als Ergebnis wurden für die Spreitung wie in Abbildung 7 dargestellt folgende Resultate gefunden:

- Für die Emollients gilt für die Spreitung folgende Reihenfolge: C < A < B.

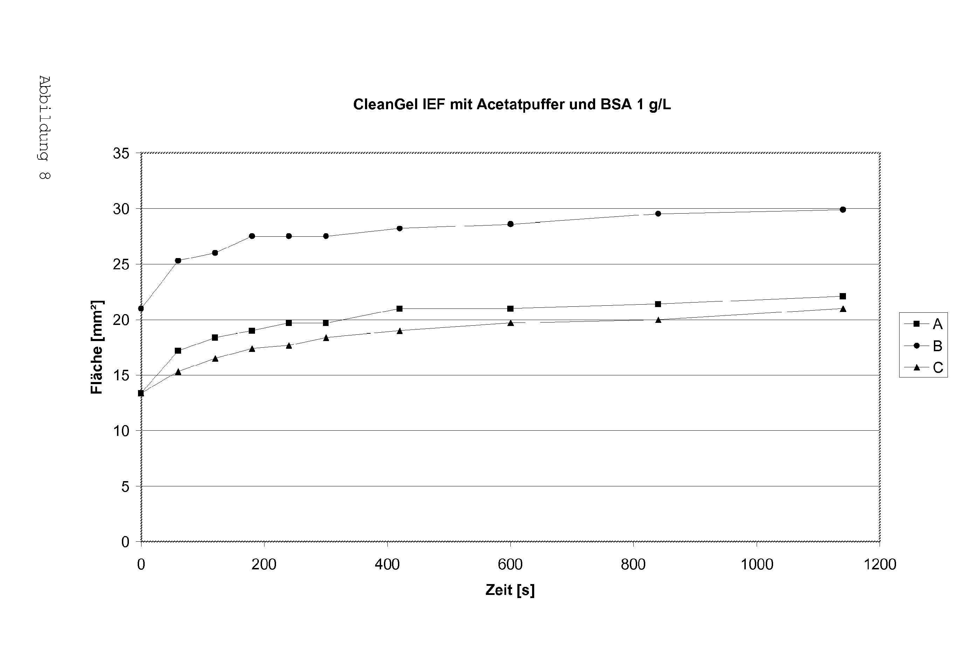

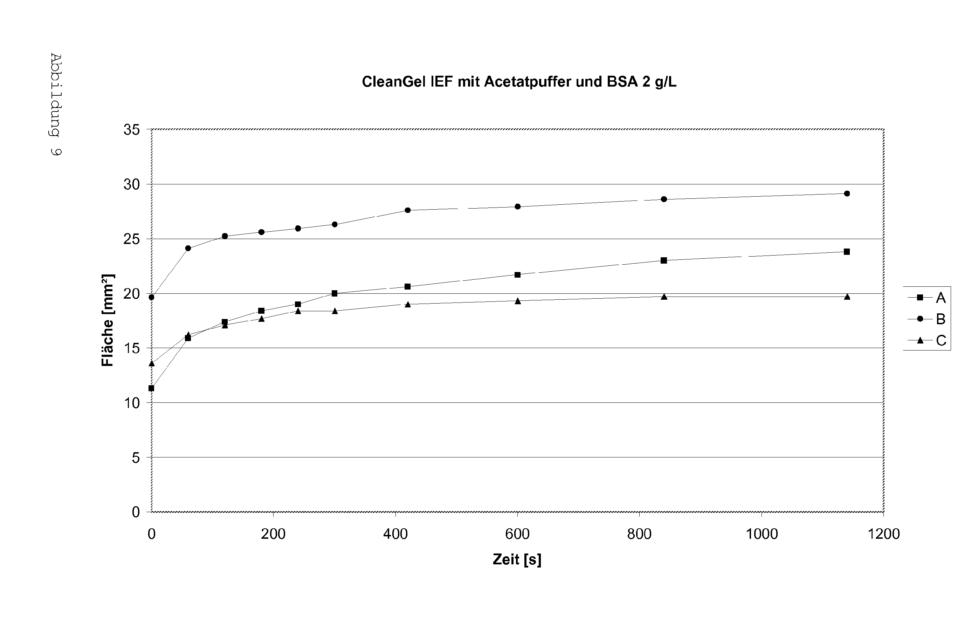

Die Spreitung der Emollients wurde wie beschrieben auf CleanGel IEF, welches mit a) 1 mg/mL und b) 2 mg/mL BSA (Albumin Fraction V, Fa. AppliChem, Art.Nr. A1391,0100, CAS-Nr. 9048-46-8) in voll entsalztem Wasser rehydratisiert worden war, durchgeführt. Für die Spreitung der Substanzen wurden wie in Abbildungen 8 und 9 dargestellt folgende Resultate gefunden:

- Für die Emollients gilt für die Spreitung für beide BSA-Konzentrationen folgende Reihenfolge: C < A < B.

Mit CleanGel IEF rehydratisiert in wässriger BSA-Lösung konnten die Ergebnisse des Paneltests für die reinen Emollients nachgestellt werden.

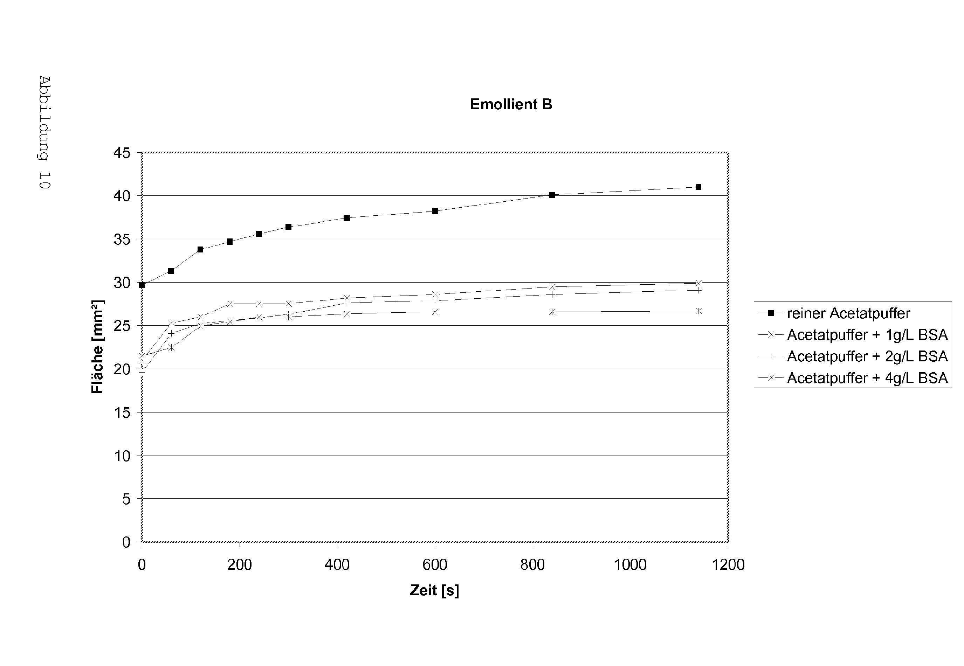

Das Spreitvermögen der Emollients konnte durch Verändern der Konzentration von BSA in den wässrigen Lösungen für die Reydratisation von CleanGel IEF beeinflußt werden (Abbildung 10).

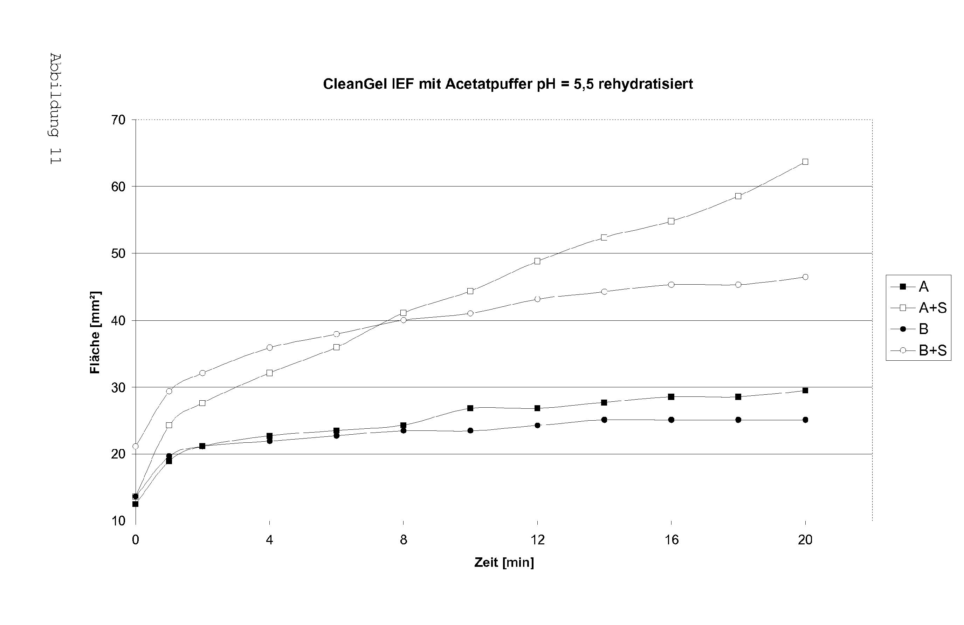

Das Spreitvermögen der Emollients konnte weiterhin durch Einstellen des pH-Wertes der Lösungen für die Reydratisation von CleanGel IEF beeinflusst werden (Abbildungen 4 und 11).

*Reihe 2*

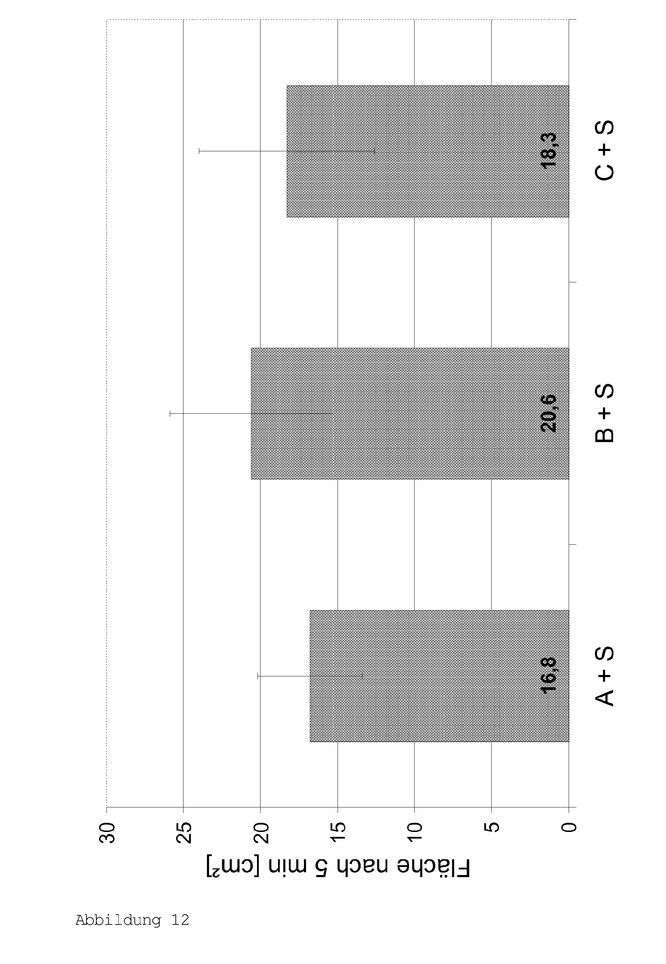

Der Paneltest wurde wie beschrieben mit 0,1%igen Mischungen von Cetyldimethicone (ABIL WAX 9840®) in den drei Emollients Decylcocoat (TEGOSOFT DC®), Bis-2-ethylhexylcarbonat (TEGOSOFT DEC®) und $C_{12}$-$C_{15}$-Alkylbenzoat (TEGOSOFT TN®), im weiteren mit A + S, B + S und C + S bezeichnet, durchgeführt. Als Ergebnis wurden für die Spreitung wie in Abbildung 12 dargestellt folgende Resultate gefunden:

- Für die Emollient-Cetyldimethiconemischungen gilt für die Spreitung: A + S ≤ C + S < B + S.

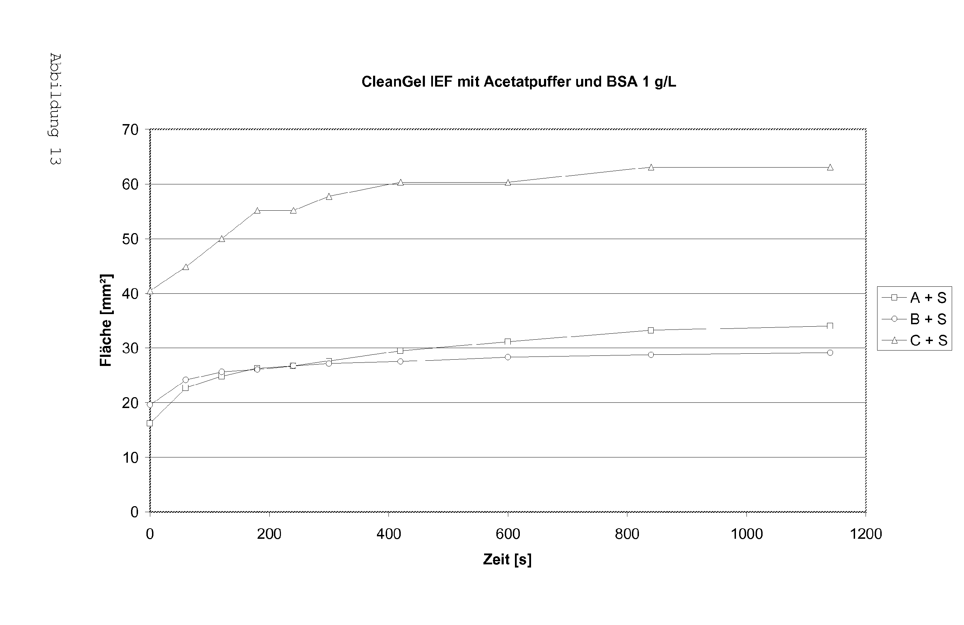

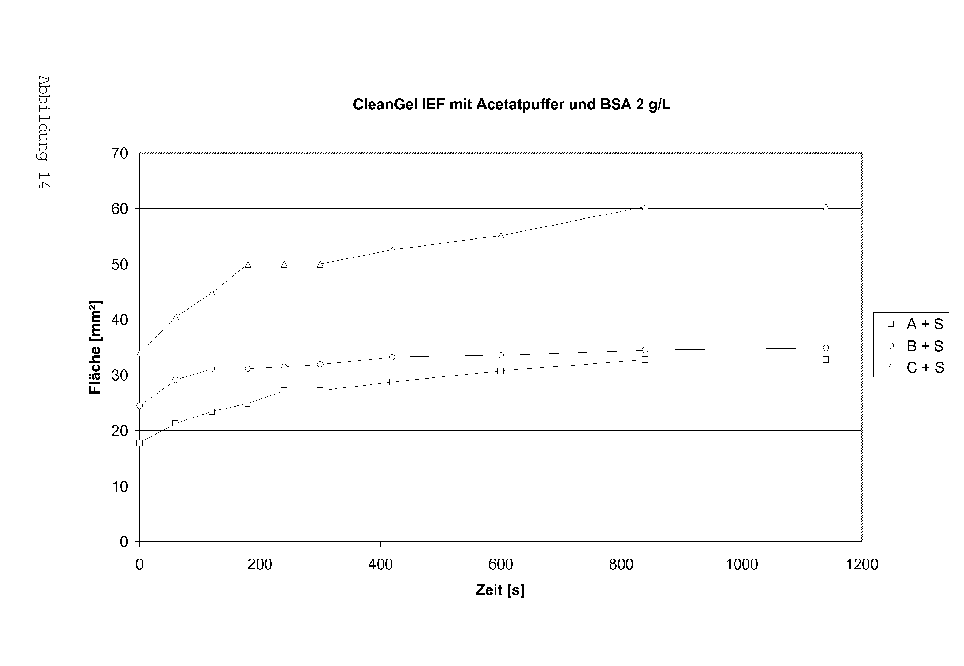

Die Spreitung der Emollient-Cetyldimethiconemischungen wurde wie beschrieben auf CleanGel IEF, welches mit a) 1 mg/mL und b) 2 mg/mL BSA (Albumin Fraction V, Fa. AppliChem, Art.Nr. A1391,0100, CAS-Nr. 9048-46-8) bei pH 7,0 rehydratisiert worden war, durchgeführt. Für die Spreitung der Substanzen wurden wie in Abbildungen 13 und 14 dargestellt folgende Resultate gefunden:

a)

- Für die Emollient-Cetyldimethiconemischungen gilt für die Spreitung: A + S ≈ B + S < C + S.

b)

- Für die Emollient-Cetyldimethiconemischungen gilt für die Spreitung: A + S < B + S < C + S.

Mit CleanGel IEF rehydratisiert in wässriger BSA-Lösung konnten die Ergebnisse des Paneltests für die Emollient-Cetyldimethiconemischungen nicht nachgestellt werden.

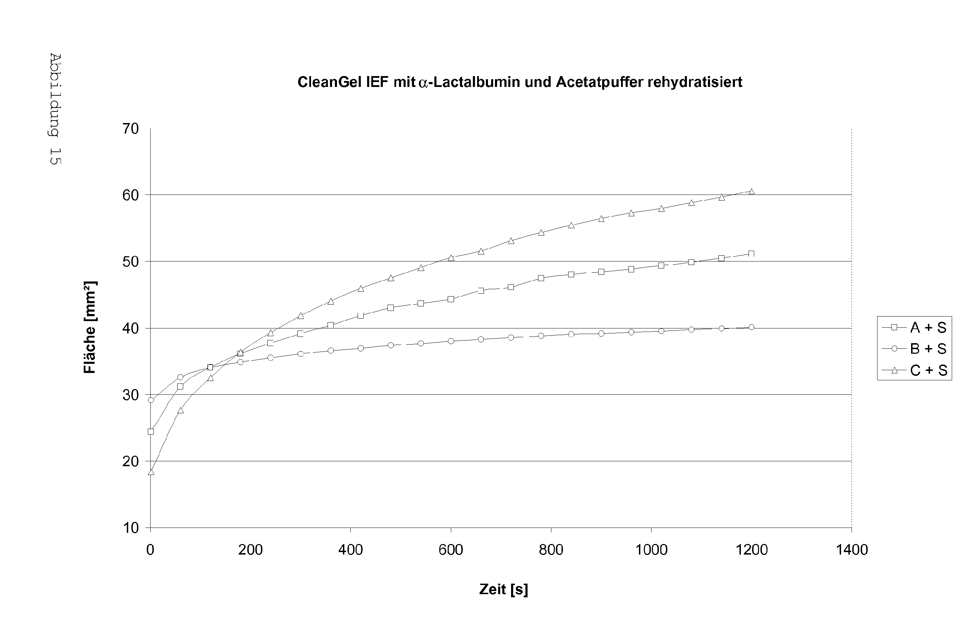

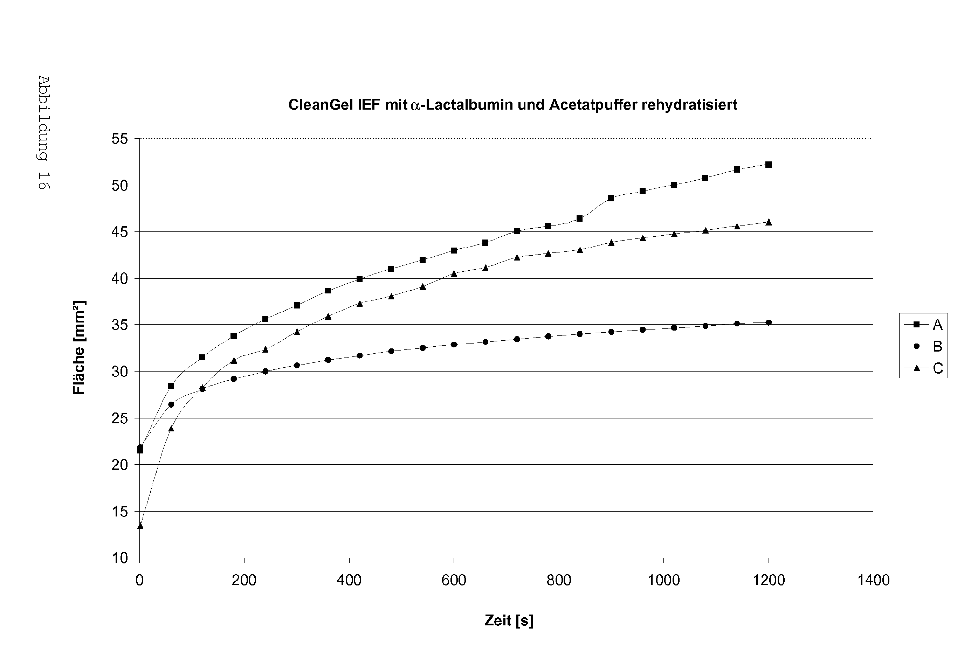

Die Spreitung a) der Emollient-Cetyldimethiconemischungen sowie b) der reinen Emollients wurde wie beschrieben auf CleanGel IEF, welches mit 150mg/L α-Lactalbumin (α-Lactalbumin aus Kuhmilch von Sigma-Aldrich, Type I, ≥85% (PAGE), lyophilisiertes Puder, CAS Nummer 9051-29-0) bei pH 5,5 (Acetatpuffer) rehydratisiert worden war, durchgeführt. Für die Spreitung der Substanzen wurden wie in Abbildungen 15 und 16 dargestellt folgende Resultate gefunden:

a)

- Für die Emollient-Cetyldimethiconemischungen gilt für die Spreitung: C + S < A + S < B + S für Spreitungszeiten, die kürzer als zwei Minuten waren, und B + S < A + S < C + S für Spreitungszeiten, die größer als zwei Minuten waren.

b)

- Für die reinen Emollients gilt für die Spreitung: B < C < A.

Mit CleanGel IEF rehydratisiert in wässriger α-Lactalbumin-Lösung konnten die Ergebnisse des Paneltests für die Emollient-Cetyldimethiconemischungen für Spreitungszeiten kleiner als zwei Minuten bedingt, für Spreitungszeiten größer als zwei Minuten nicht und für die reinen Emollients ebenfalls nicht nachgestellt werden.

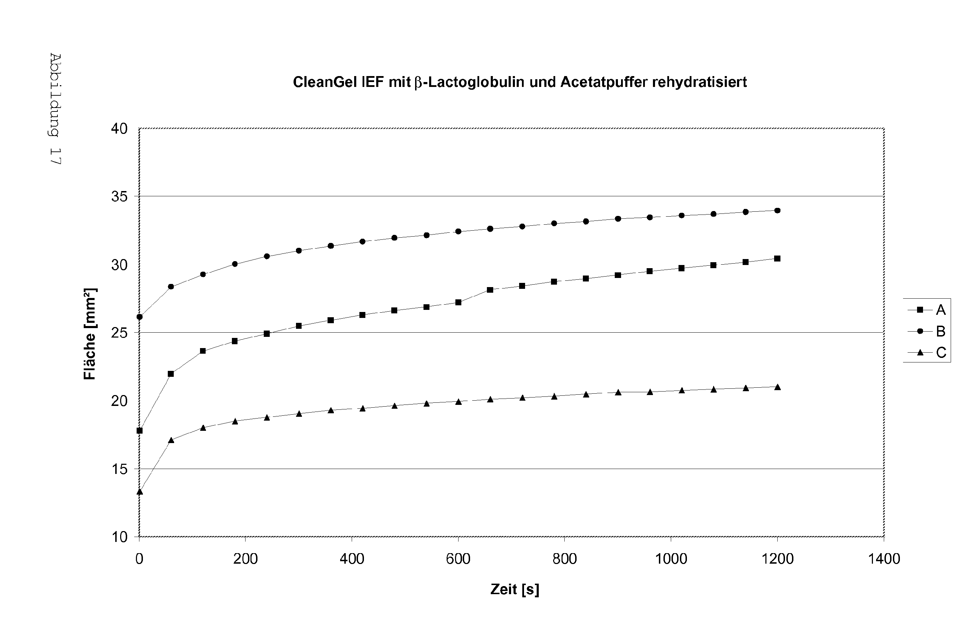

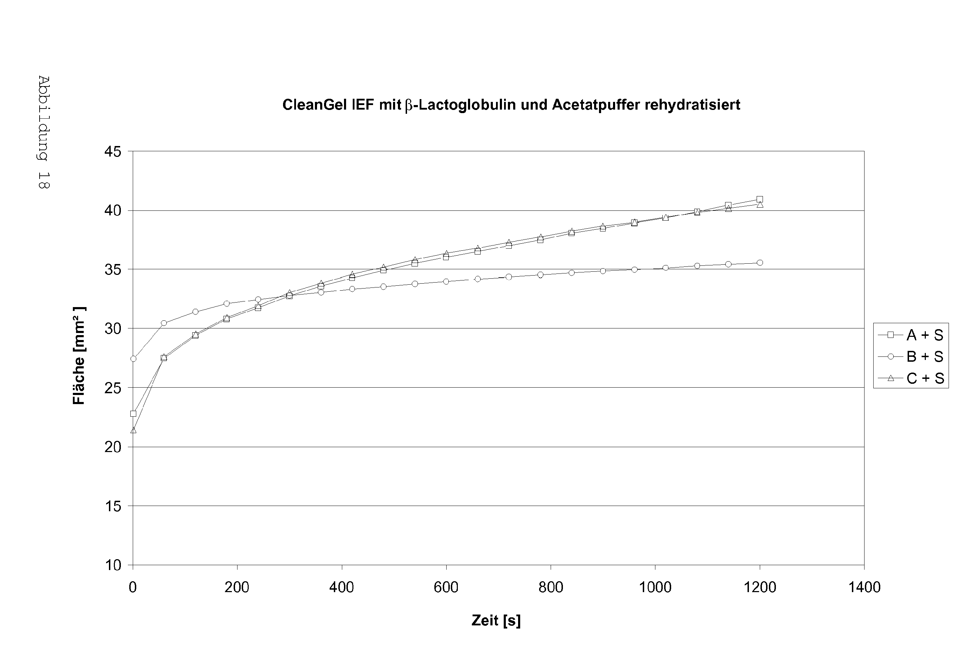

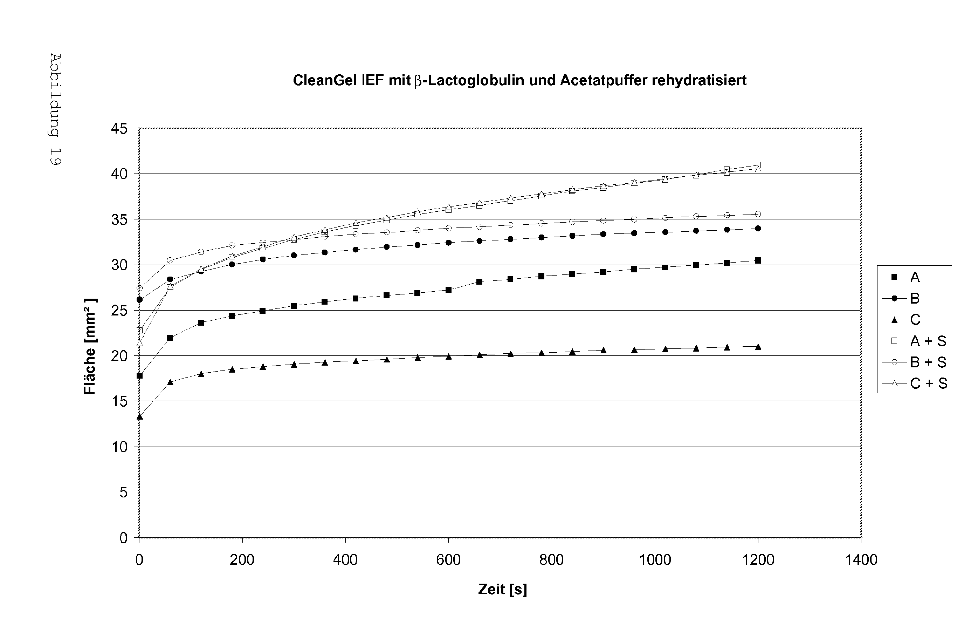

Die Spreitung a) der reinen Emollients sowie b) der Emollient-Cetyldimethiconemischungen wurde wie beschrieben auf CleanGel IEF, welches mit 150mg/L β-Lactoglobulin (β-Lactoglobulin aus Kuhmilch, Sigma-Aldrich, ~90% (PAGE), lyophilisiertes Puder, CAS Number 9045-23-2) bei pH 5,5 (Acetatpuffer) rehydratisiert worden war, durchgeführt. Für die Spreitung der Substanzen wurden wie in Abbildungen 17 bis 19 dargestellt folgende Resultate gefunden:

- Für die Emollients gilt für die Spreitung folgende Reihenfolge: C < A < B.
- Für die Emollient-Cetyldimethiconemischungen gilt für die Spreitung: A < A + S, B < B + S und C < C + S. Weiterhin gilt innerhalb der ersten fünf Minuten folgende Reihenfolge: A + S ≤ C + S < B + S.
- Weiterhin gilt, dass die Spreitung von Emollient B als einziges in der Größenordnung der übrigen Emollients liegt.

Mit CleanGel IEF rehydratisiert in wässriger β-Lactoglobulin-Lösung konnten die Ergebnisse des Paneltests für die reinen Emollients und für die Emollient-Cetyldimethiconemischungen somit nachgestellt werden.

## Patentansprüche

1. Vorrichtung umfassend einen Hautersatzstoff zur Untersuchung des Verhaltens von topisch anzuwendenden, kosmetischen und/oder pharmazeutischen Kompositionen, wobei der Hautersatzstoff eine dreidimensional vernetzte Polyacrylamid-Matrix ist,
   **dadurch gekennzeichnet,**
   **dass** in besagter Matrix Wasser und mindestens ein Polypeptid eingebunden ist.

2. Vorrichtung gemäß Anspruch 1, **dadurch gekennzeichnet, dass** die Polyacrylamid-Matrix einen T-Wert von T = 3 bis 15 % und einen C-Wert von C = 2 bis 6 % aufweist.

3. Vorrichtung gemäß Anspruch 1, **dadurch gekennzeichnet, dass** die Polyacrylamid-Matrix einen T-Wert von T = 4 bis 12 % und einen C-Wert von C = 2 bis 4 % aufweist.

4. Vorrichtung gemäß Anspruch 1, **dadurch gekennzeichnet, dass** die Polyacrylamid-Matrix einen T-Wert von T = 5 % und einen C-Wert von C = 3 % aufweist.

5. Vorrichtung gemäß zumindest einem der Ansprüche 1 bis 4, **dadurch gekennzeichnet, dass** das Polypeptid ein Albumin oder Globulin ist.

6. Vorrichtung gemäß zumindest einem der Ansprüche 1 bis 5, **dadurch gekennzeichnet, dass** das Polypeptid Bovines Serum Albumin ist.

7. Vorrichtung gemäß zumindest einem der Ansprüche 1 bis 5, **dadurch gekennzeichnet, dass** das Polypeptid beta-Lactoglobulin ist.

8. Vorrichtung gemäß zumindest einem der Ansprüche 1 bis 7, **dadurch gekennzeichnet, dass** die Vorrichtung eine digitale Kamera aufweist, die an einen Rechner mit einer Auswertesoftware angeschlossen ist.

9. Vorrichtung gemäß zumindest einem der Ansprüche 1 bis 8, **dadurch gekennzeichnet, dass** die für die automatische Auswertung notwendige Kontrastbildung durch Lichtbrechung innerhalb des Tropfens durch Anstrahlen eines weißen Ausschnittes in der Größe des Substrates innerhalb einer schwarzen Fläche unterhalb des Substrates mittels einer Hauptlichtquelle erzielt wird.

10. Verfahren zur Anpassung einer Vorrichtung nach Anspruch 1 an eine Reihe zu untersuchender Kompositionen, **gekennzeichnet durch** die Verfahrensschritte:

A. Bestimmung der Parameter $C_1$ und n des Spreitverhaltens der zu untersuchenden Kompositionen im Rahmen eines Paneltests,
B. Variieren der C- und T-Werte der Polyacrylamid-Matrix, des Polypeptides und/oder seiner Konzentration und/oder des pH-Wertes und/oder der Art des Einbringens des Polypeptides in die Matrix unter Erhalt verschiedener Ausführungsformen und Bestimmung der Parameter $C_1$ und n des Spreitverhaltens der zu untersuchenden Kompositionen für jede Ausführungsform, und
C. Vergleichen der Parameter aus A und B und Auswahl der Ausführungsform der Vorrichtung mit der Polyacrylamid-Matrix, die die größte Korrelation der Messdaten zeigt.

11. Verwendung einer Vorrichtung gemäß zumindest einem der Ansprüche 1 bis 9 zur Simulation oder Vorhersage des Spreitverhaltens von kosmetischen und/oder pharmazeutischen Formulierungen auf menschlicher Haut.

y = 0,0228x + 1,1429
lg Fläche
1,30
1,28
1,26
1,24
1,22
1,20
1,18
1,16
1,14
1,12
1,10
lg Zeit
0
0,5
1
1,5
2
2,5
3
3,5
4

Abbildung 1

Fläche [mm²]
20
19
18
17
16
15
14
13
12
11
10
0
100
200
300
400
500
600
Zeit [s]

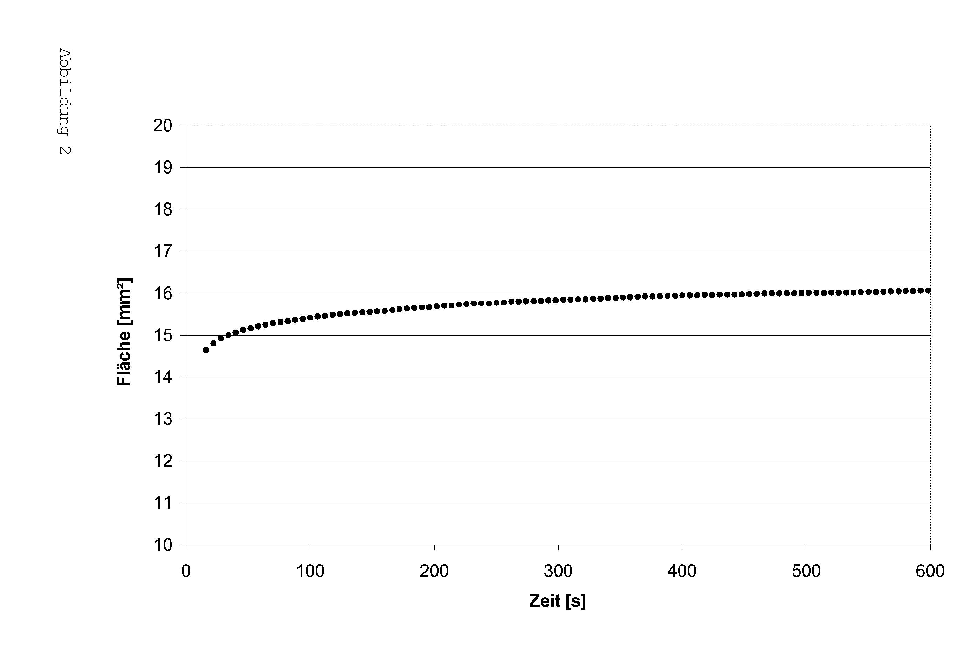

Abbildung 2

Emollient
Emollient + Additiv S
Fläche nach 5 min [cm²]
0
5
10
15
20
25
30
7,5
16,8
14,5
20,6
4,8
18,3
A
B
C

Abbildung 3

CleanGel IEF mit VE-Wasser rehydratisiert
Fläche [mm²]
Zeit [min]
A
B
A+S
B+S
10
20
30
40
50
60
0
4
8
12
16
20

Abbildung 4

CleanGel IEF mit Lipidmischung B rehydratisiert
Fläche [mm²]
Zeit [s]
A
B
C
0
5
10
15
20
25
0
200
400
600
800
1000
1200

Abbildung 5

CleanGel IEF mit Lipidmischung M 3,5
Fläche [cm²]
Zeit [s]
0
1
2
3
4
5
6
0
200
400
600
800
1000
1200
A
A+S
B
B+S
C
C+S

Abbildung 6

Fläche nach 5 min [cm²]
25
20
15
10
5
0
7,5
14,5
4,8
A
B
C


Abbildung 7

CleanGel IEF mit Acetatpuffer und BSA 1 g/L
Fläche [mm²]
Zeit [s]
0
5
10
15
20
25
30
35
0
200
400
600
800
1000
1200
A
B
C

Abbildung 8

CleanGel IEF mit Acetatpuffer und BSA 2 g/L
Fläche [mm²]
Zeit [s]
A
B
C
0
5
10
15
20
25
30
35
0
200
400
600
800
1000
1200

Abbildung 9

Emollient B
reiner Acetatpuffer
Acetatpuffer + 1g/L BSA
Acetatpuffer + 2g/L BSA
Acetatpuffer + 4g/L BSA
Fläche [mm²]
Zeit [s]
45
40
35
30
25
20
15
10
5
0
200
400
600
800
1000
1200

Abbildung 10

CleanGel IEF mit Acetatpuffer pH = 5,5 rehydratisiert
A
A+S
B
B+S
Fläche [mm²]
70
60
50
40
30
20
10
Zeit [min]
0
4
8
12
16
20

Abbildung 11

Fläche nach 5 min [cm²]
0
5
10
15
20
25
30
16,8
20,6
18,3
A + S
B + S
C + S


Abbildung 12

CleanGel IEF mit Acetatpuffer und BSA 1 g/L
Fläche [mm²]
Zeit [s]
A + S
B + S
C + S
0
10
20
30
40
50
60
70
0
200
400
600
800
1000
1200

Abbildung 13

CleanGel IEF mit Acetatpuffer und BSA 2 g/L
A + S
B + S
C + S
Fläche [mm²]
70
60
50
40
30
20
10
0
Zeit [s]
0
200
400
600
800
1000
1200

Abbildung 14

CleanGel IEF mit α-Lactalbumin und Acetatpuffer rehydratisiert
Fläche [mm²]
Zeit [s]
A + S
B + S
C + S
10
20
30
40
50
60
70
0
200
400
600
800
1000
1200
1400

Abbildung 15

CleanGel IEF mit α-Lactalbumin und Acetatpuffer rehydratisiert
Fläche [mm²]
Zeit [s]
A
B
C
55
50
45
40
35
30
25
20
15
10
0
200
400
600
800
1000
1200
1400

Abbildung 16

CleanGel IEF mit β-Lactoglobulin und Acetatpuffer rehydratisiert
A
B
C
Fläche [mm²]
40
35
30
25
20
15
10
0
200
400
600
800
1000
1200
1400
Zeit [s]

Abbildung 17

CleanGel IEF mit β-Lactoglobulin und Acetatpuffer rehydratisiert
A + S
B + S
C + S
Fläche [mm²]
45
40
35
30
25
20
15
10
0
200
400
600
800
1000
1200
1400
Zeit [s]

Abbildung 18

CleanGel IEF mit β-Lactoglobulin und Acetatpuffer rehydratisiert
Fläche [mm²]
Zeit [s]
0
5
10
15
20
25
30
35
40
45
0
200
400
600
800
1000
1200
1400
A
B
C
A + S
B + S
C + S

Abbildung 19

Europäisches Patentamt
European Patent Office
Office européen des brevets

# EUROPÄISCHER RECHERCHENBERICHT

Nummer der Anmeldung
EP 08 16 9840

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | US 2002/182658 A1 (POLAK ANTHONY J [US]; BALLERSTADT RALPH [US]; BEUHLER ALLYSON [US]; G) 5. Dezember 2002 (2002-12-05)<br>* Absatz [0034] - Absatz [0037] *<br>* Absatz [0046] *<br>* Anspruch 1 *<br>* Ansprüche 11-15 *<br>----- | 1,5<br>2-4,7-11 | INV.<br>A61L27/60<br>C12N5/00 |
| X<br>A | US 2003/006143 A1 (BANERJEE SUKANTA [US]; PODUAL KAIRALI [US]; SEUL MICHAEL [US]) 9. Januar 2003 (2003-01-09)<br>* Absatz [0051] *<br>* Absatz [0055] - Absatz [0057] *<br>* Absatz [0061] *<br>----- | 1<br>2-11 | |
| X<br>A | EP 1 764 147 A1 (TNO [NL]) 21. März 2007 (2007-03-21)<br>* Absatz [0023] *<br>* Absatz [0042] - Absatz [0044] *<br>* Ansprüche 1-8 *<br>----- | 1<br>2-11 | |
| | | | RECHERCHIERTE SACHGEBIETE (IPC) |
| X<br>A | JP 59 098690 A (TOKYO SHIBAURA ELECTRIC CO) 7. Juni 1984 (1984-06-07)<br>* Zusammenfassung *<br>----- | 1<br>2-11 | A61L<br>C12N |
| X<br>A | JP 04 135565 A (TERUMO CORP) 11. Mai 1992 (1992-05-11)<br>* Zusammenfassung *<br>----- | 1<br>2-11 | |
| X,P | EP 1 965 212 A1 (UNIV HOKKAIDO NAT UNIV CORP [JP]; SEIKEN CO [JP]; MATSUDA TAKEHIDE [J)<br>3. September 2008 (2008-09-03)<br>* Absätze [0032], [0033] *<br>* Ansprüche 1-3 *<br>* Ansprüche 9,11 *<br>----- | 1 | |

-/--

Der vorliegende Recherchenbericht wurde für alle Patentansprüche erstellt

| Recherchenort | Abschlußdatum der Recherche | Prüfer |
|---|---|---|
| München | 16. September 2009 | Michalitsch, Richard |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

1

EPO FORM 1503 03.82 (P04C03)

Europäisches Patentamt
European Patent Office
Office européen des brevets

# EUROPÄISCHER RECHERCHENBERICHT

Nummer der Anmeldung
EP 08 16 9840

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | US 2005/167269 A1 (UPDYKE TIMOTHY V [US]; ENGELHORN SHELDON C [US]) 4. August 2005 (2005-08-04) * das ganze Dokument * ----- | 1 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 16. September 2009 | Michalitsch, Richard |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 08 16 9840

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-09-2009

| Im Recherchenbericht angeführtes Patentdokument | Datum der Veröffentlichung | Mitglied(er) der Patentfamilie | Datum der Veröffentlichung |
|---|---|---|---|
| US 2002182658 A1 | 05-12-2002 | KEINE | |
| US 2003006143 A1 | 09-01-2003 | AU 2002367464 A1 | 29-09-2003 |
| | | CA 2449040 A1 | 25-09-2003 |
| | | WO 03079401 A2 | 25-09-2003 |
| | | US 2003138842 A1 | 24-07-2003 |
| | | US 2006068447 A1 | 30-03-2006 |
| | | US 2007248993 A1 | 25-10-2007 |
| EP 1764147 A1 | 21-03-2007 | WO 2007035086 A1 | 29-03-2007 |
| | | US 2008290021 A1 | 27-11-2008 |
| JP 59098690 A | 07-06-1984 | KEINE | |
| JP 4135565 A | 11-05-1992 | KEINE | |
| EP 1965212 A1 | 03-09-2008 | WO 2007052613 A1 | 10-05-2007 |
| | | US 2009142781 A1 | 04-06-2009 |
| US 2005167269 A1 | 04-08-2005 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9721097 A
- US 4877454 A
- US 5015431 A


### In der Beschreibung aufgeführte Nicht-Patentliteratur


- **SAMBROOK et al.** Molecular Cloning: A Laboratory Manual. Cold Spring Harbor Laboratory, 1989